# NEUE EUROPÄISCHE PATENTSCHRIFT

(11) **EP 1 294 402 B2**
(45) Veröffentlichungstag und Bekanntmachung des Hinweises auf die Entscheidung über den Einspruch: **12.08.2009**
(45) Hinweis auf die Patenterteilung: 11.01.2006
(21) Anmeldenummer: 01943329.1
(22) Anmeldetag: 08.05.2001
(51) Int. Cl.: A61K 47/20, A61K 31/565, A61K 31/566, A61K 31/568, A61K 31/49, A61K 31/4045, A61P 43/00

(54) **VERBINDUNGEN MIT EINER SULFONAMIDGRUPPE UND DIESE VERBINDUNGEN ENTHALTENDE PHARMAZEUTISCHE ZUSAMMENSETZUNGEN**
COMPOUNDS WITH A SULPHONAMIDE GROUP AND PHARMACEUTICAL COMPOSITIONS CONTAINING THESE COMPOUNDS
COMPOSES AYANT UN GROUPE SULFONAMIDE ET COMPOSITIONS PHARMACEUTIQUES CONTENANT CES COMPOSES

(30) Priorität: 31.05.2000 DE 10027887
(43) Veröffentlichungstag der Anmeldung: 26.03.2003
(73) Patentinhaber: Bayer Schering Pharma Aktiengesellschaft, 13353 Berlin (DE)
(72) Erfinder: ELGER, Walter, 14195 Berlin (DE); HILLISCH, Alexander, 07743 Jena (DE); HEDDEN, Annemarie, 22393 Hamburg (DE); SCHWARZ, Sigfrid, 07743 Jena (DE); SCHÖLLKOPF, Klaus, 13467 Berlin (DE)
(74) Vertreter: Weiss, Wolfgang
(86) Internationale Anmeldenummer: PCT/EP2001/005169
(87) Internationale Veröffentlichungsnummer: WO 2001/091797

(56) Entgegenhaltungen:
- WO-A-00/06475
- WO-A-01/51055
- WO-A-93/05064
- WO-A-94/14827
- WO-A-94/26702
- WO-A-96/05216
- WO-A-97/14712
- DE-A1- 19 712 488
- DE-B- 1 203 042
- US-A- 4 513 006
- US-A- 4 792 569
- US-A- 5 025 031
- US-A2- 5 001 234
- BARTH A ET AL: "INFLUENCE OF SUBCHRONIC ADMINISTRATION OF OESTRADIOL, ETHINYLOESTRADIOL AND OESTRADIOL SULPHAMATE ON BILE FLOW, BILE ACIDEXCRETION, AND LIVER AND BILIARY GLUTATHIONE STATUS IN RATS" ARCHIVES OF TOXICOLOGY, SPRINGER VERLAG, DE, Bd. 71, Nr. 7, 1997, Seiten 443-449, XP000986430 ISSN: 0340-5761
- ELGER W ET AL: "SULFAMATES OF VARIOUS ESTROGENS ARE PRODRUGS WITH INCREASED SYSTEMIC AND REDUCED HEPATIC ESTROGENICITY AT ORAL APPLICATION" JOURNAL OF STEROID BIOCHEMISTRY AND MOLECULAR BIOLOGY, ELSEVIER SCIENCE LTD., OXFORD, GB, Bd. 55, Nr. 3/4, 1995, Seiten 395-403, XP000618892 ISSN: 0960-0760
- MARYANOFF B E ET AL: "ANTICONVULSANT SUGAR SULFAMATES. POTENT CYCLIC SULFATE AND CYCLIC SULFITE ANALOGUES OF TOPIRAMATE" BIOORGANIC & MEDICINAL CHEMISTRY LETTERS, OXFORD, GB, Bd. 3, Nr. 12, 1993, Seiten 2653-2656, XP000914306 ISSN: 0960-894X
- J MED CHEM Bd. 38, Nr. 13, 23 Juni 1995, Seiten 2286 - 2291

## Beschreibung

Die vorliegende Erfindung betrifft Verbindungen zur pharmatentisiven verwendung, die als Prodrug und/oder Träger die Aufnahme eines Wirkstoffs in die Erythrozyten und/oder die Bindung eines Wirkstoffs an die Erythrozyten ermöglichen.

Ziel der vorliegenden Erfindung ist, Wirkstoffe, wie körpereigene Substanzen, Naturstoffe und synthetische Substanzen mit therapeutisch wertvollen Eigenschaften mit hohem "first pass" Effekt sinnvoll oral verfügbar zu machen bzw. bezüglich oraler Aktivität entscheidend zu verbessern.

Dieses Ziel wird erreicht, indem man eine gegebenenfalls substituierte Sulfonamidgruppe, sozusagen als "Anker", in den Wirkstoff einbaut, der die Aufnahme in bzw. die Bindung an die Erythrozyten ermöglicht.

Das heißt, die vorliegende Erfindung betrifft Verbindungen zur pharmatentisiven verwendung, die als Prodrug und/oder Träger die Aufnahme eines Wirkstoffs in die Erythrozyten und/oder die Bindung eines Wirkstoffs an die Erythrozyten ermöglichen, wobei die Aufnahme der Verbindungen in und/oder die Bindung der Verbindungen an die Erythrozyten durch eine Gruppe

- SO₂NR¹R²

ermöglicht wird, wobei R¹ und R² unabhängig voneinander ein Wasserstoffatom, eine Acylgruppe, eine Alkylgruppe, eine Cycloalkylgruppe, eine Arylgruppe, eine Cyanogruppe oder eine Hydroxygruppe bedeuten.

Bevorzugt bedeutet einer der Reste R¹ und R² ein Wasserstoffatom, besonders bevorzugt bedeuten beide Reste R¹ und R² ein Wasserstoffatom.

Die N-mono-substituierten bzw. N,N-disubstituierten Verbindungen der Erfindung können einen oder zwei N-Alkyl-, N-Alkenyl-, N-Cycloalkyl, N-Acyl- oder N-Arylsubstituenten aufweisen, die jeweils vorzugsweise bis zu 10 Kohlenstoffatome aufweisen. R¹ und/oder R² bedeuten insbesondere eine Alkyl- oder Acylgruppe mit 1 bis 6 Kohlenstoffatomen. Beispiele sind eine Methyl-, Ethyl-, Propyl- oder iso-Propylgruppe bzw. die entsprechenden Acylderivate. Beispiele für eine Arylgruppe sind eine Phenyl- bzw. eine Tolylgruppe. Beispiele für eine Cycloalkylgruppe sind eine Cyclopentyl- oder eine Cyclohexylgruppe.

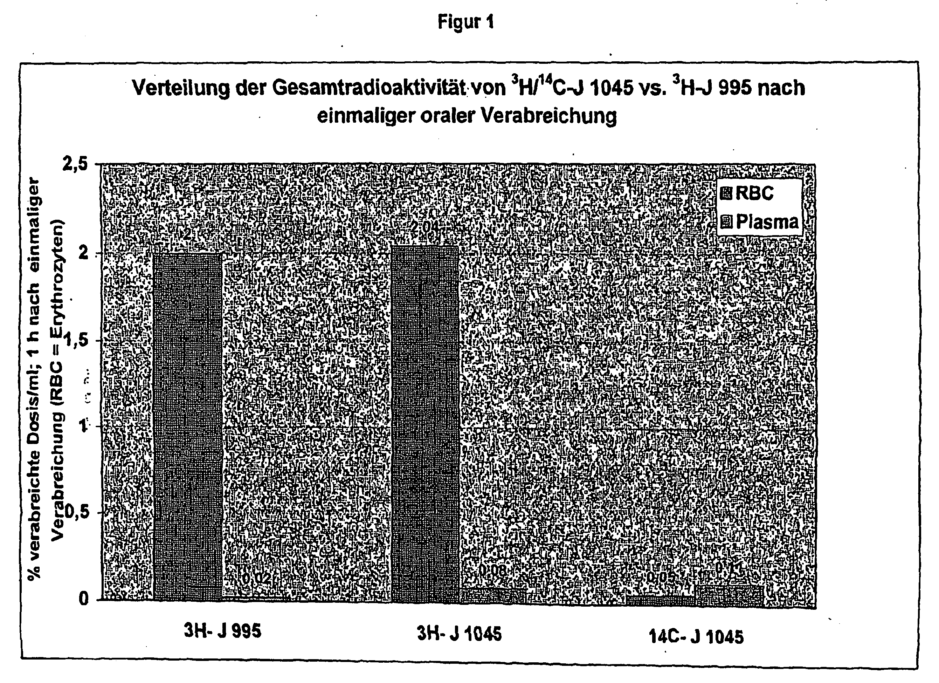

Es gibt Hinweise darauf, daß bei oraler Verabreichung die beiden Reste R¹ und R², wenn sie verschieden von einem Wasserstoffatom sind, abgespalten und durch ein Wasserstoffatom ersetzt werden, so daß in diesem Fall nicht die Gruppe - SO₂NR¹R², sondern die Gruppe - SO₂NH₂ für die Aufnahme in bzw. Bindung an die Erythrozyten sorgt. Figur 1 ist eine Diagramm, das die unterschiedliche Aktivitätsverteilung für Tritium um ¹⁴C auf Erythrozyten und Plasma nach oraler Applikation von 17β-Hydroxy-estra-1,3,5(10)-trien-3-yl-(N-acetyl)sulfamat (J1045, ¹⁴C-markierter Acylsubstituent und ³H-markiertes Steroidgerüst) zeigt. Wie aus der Abbildung ersichtlich, ist der ¹⁴C-markierte Acylsubstituent des Sulfamatrestes nach oraler Applikation im Blut nicht mehr vorhanden. Ferner belegen die Daten, daß erfindungsgemäße Substanzen im Erythrozyten-Kompartment gegenüber dem Plasma angereichert werden.

Die erfindungsgemäß bevorzugte Sulfonamidgruppe kann also auch erst im Körper aus N-monosubstituierten bzw. N,N-disubstituierten Sulfonamidgruppen, beispielsweise N-Acylsulfonamiden, gebildet werden.

Erfindungsgemäß wird unter Prodrug (Pro-Pharmakon) ein selbst biologisch weitgehend inaktiver Arzneistoff verstanden, der erst im Körper zum eigentlichen Wirkstoff umgesetzt wird. Das Prodrug kann zusätzlich zu den Wirkungen des Wirkstoffs weitere pharmakologische Wirkungen aufweisen. Wenn diese Wirkungen *überwiegend* oder *ganz* die Therapie-relevanten Wirkungen sind, sind die erfindungsgemäßen Verbindungen in Grenzfällen selbst auch als Wirkstoff und nicht nur als Prodrug zu betrachten ("Träger").

Die Aufnahme der Verbindungen in und/oder die Bindung der Verbindungen an die Erythrozyten erfolgt über Hämoglobin, Membranproteine und/oder Carboanhydrase, das heißt, die Carboanhydrase I (hCAI) und II (hCAII).

Durch die Aufnahme der Verbindungen in die Erythrozyten und/oder durch die Bindung der Verbindungen an die Erythrozyten wird ein Depot des Wirkstoffs in den Erythrozyten gebildet, wobei ein wesentlicher Teil des Wirkstoffs im Körper in Erythrozyten vorliegt.

Erfindungsgemäß wird unter dem Begriff "Depotbildung" verstanden, daß die Verbindung (bzw. der Wirkstoff) in den Erythrozyten um den Faktor 10 bis 1000, vorzugsweise 20 bis 1000, besonders bevorzugt 30 bis 1000, über Plasmaspiegel angereichert ist, wobei der Faktor nach Trennung von Erythrozyten und Plasma durch Zentrifugation und die Ermittlung der Konzentration der erfindungsgemäßen Substanz in Erythrozyten und Plasma bestimmt wird. Für Estradiolsulfamat (J995) (nicht Gegenstand der Erfindung) wurde gefunden, daß der oben genannte Faktor zu beliebigen Zeitpunkten 98:2 beträgt.

Grundsätzlich kann es sich bei den Verbindungen um Verbindungen handeln, die in den Erythrozyten ihre Wirkung entfalten. Bevorzugt verhindern die Verbindungen und/oder der in den Verbindungen enthaltene Wirkstoff in diesem Fall den Parasitenbefall der Erythrozyten, der beispielsweise bei der Malaria ein wesentlicher Aspekt der Krankheit ist

Die Verbindungen weisen die folgende Struktur auf: 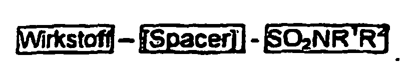 wobei R¹ und R² die vorstehend gegebene Bedeutung haben und der 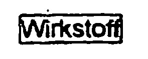 in seiner freien Form mindestens eine 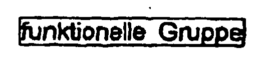 aufweist, wobei bevorzugt mindestens einer der Reste R¹ und R² ein Wasserstoffatom bedeutet und besonders bevorzugt beide ein Wasserstoffatom bedeuten.

Wenn die Verbindungen als Prodrug wirken, wird die therapeutisch erwünschte Wirkung durch Freisetzung, insbesondere hydrolytische Spaltung, des im Prodrug enthaltenen Wirkstoffs und/oder seiner Metabolite erhalten. Für J995 (nicht Gegenstand der Erfindung) wurde gefunden, daß diese Verbindung in den Erythrozyten stabil ist, und erst im Plasma oder in Geweben die Freisetzung des Wirkstoffs aus dem Prodrug erfolgt.

Die funktionelle Gruppe ist bevorzugt eine Gruppe - COOH oder eine von dieser Gruppe abgeleitete Gruppe, wie eine Estergruppe, die zusammen mit dem Spacer und mit der Gruppe - SO₂NH₂ eine Gruppe - C(O) - Spacer - SO₂NH₂ bildet, wobei der Spacer in diesem Fall eine Gruppe - A - B - (O)ₛ - ist, wobei s eine Zahl 0 oder 1 ist, A für S, O oder NR³ steht, wobei R³ ein Wasserstoffatom, eine Alkylgruppe, bevorzugt mit 1 bis 4 Kohlenstoffatomen, oder eine Acylgruppe, bevorzugt mit 1 bis 4 Kohlenstoffatomen, ist, und B aus einer Alkylengruppe, einer Arylengruppe, einer Alkylenarylengruppe oder einer Alkylenarylenalkylengruppe ausgewählt ist, die gegebenenfalls substituiert sind.

Die funktionelle Gruppe ist ferner bevorzugt eine Gruppe - YH, die zusammen mit dem Spacer und mit der Gruppe - SO₂NH₂ eine Gruppe - Y - Spacer - SO₂NH₂ bildet, wobei Y für S, O oder NR⁴ steht, wobei R⁴ ein. Wasserstoffatom, ein Acyl-, bevorzugt mit 1 bis 4 Kohlenstoffatomen, oder eine Alkylgruppe, bevorzugt mit 1 bis 4 Kohlenstoffatomen, ist oder NR⁴ Teil eines heterocyclischen Ringsystems ist, wobei der Spacer eine Gruppe 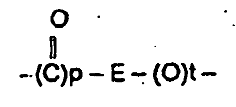 ist, wobei t und p eine Zahl 0 oder 1 sind und E aus einer Alkylengruppe, einer Arylengruppe, einer Alkylenarylengruppe oder einer Alkylenarylenalkylengruppe ausgewählt ist, die gegebenenfalls substituiert sind, oder,
wobei der Spacer eine Gruppe 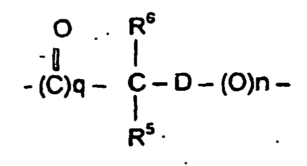 bedeutet, wobei n und q eine Zahl 0 oder 1 sind, R⁵ und R⁶ unabhängig voneinander ein Wasserstoffatom oder eine Alkylgruppe, bevorzugt mit 1 bis 4 Kohlenstoffatomen, bedeuten und D eine Arylengruppe, insbesondere eine Phenylengruppe, bedeutet, oder wobei der Spacer eine Gruppe 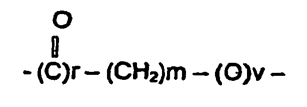 bedeutet, wobei r und v eine Zahl 0 oder 1 sind und m eine Zahl von 1 bis 15 bedeutet.

Die Struktur der erfindungsgemäßen Verbindungen wurde aus Gründen der Einfachheit anhand der Gruppe - SO₂NH₂ erläutert. Das für die Gruppe - SO₂NH₂ Gesagte gilt entsprechend für die Gruppe - SO₂NR¹R².

Der Wirkstoff kann grundsätzlich jeder Wirkstoff sein, der mindestens eine der vorstehend genannten funktionellen Gruppen aufweist und mittels dieser über einen Spacer an die Gruppe - SO₂NR¹R² gebunden ist.

Der Wirkstoff wird bevorzugt aus Androgenen, Anabolika, Antiandrogenen, Estrogenen, Gestagenen, Glucocorticoiden, Amöbiziden, Anti-Diuretika, Antigonatropinen, Ulcus-Therapeutika, Neuropharmaka, Dopaminrezeptorantagonisten, Dopamin, Apomorphin, Melatonin und Peptiden, wie GnRH (Gonatropin Releasing Hormone) und anderen hypothalamisch, regulatorisch aktiven Peptiden, ausgewählt

Bevorzugt ist der Wirkstoff ein Androgen, wie Testosteron, wobei die funktionelle Gruppe die 17-Hydroxygruppe oder die 3-Carbonylgruppe des Androgens ist.

Bevorzugt ist der Wirkstoff ein Estrogen, wie Estradiol, Estriol oder Estron, wobei die funktionelle Gruppe eine 3-, 16- oder 17-Hydroxygruppe oder 17-Carbonylgruppe ist.

Bevorzugt ist der Wirkstoff ein Gestagen, wie Norethisteron, Dienogest, Drospirenon oder Levonorgestrel, wobei die funktionelle Gruppe eine 17-Hydroxygruppe oder eine 3-Carbonylgruppe ist.

Besonders bevorzugt ist der Wirkstoff ein Anti-Malariamittel, wie Arteether, Artemether, Artesunat, Chloroquin, Pamaquin, Primaquin, Pyrethamin, Mefloquin, Proguanil, Chinchonidin, Cinchonin, Hydroxychloroquin, Pamaquin, Primaquin, Pyrimethamin sowie Chinin oder ein Chinin-Derivat, wie Chinin-Bisulfat, Chinin-Carbonat, Chinin-Dihydrobromid, Chinin-Dihydrochlorid, Chinin-Ethylcarbonat, Chinin-Format, Chinin-Gluconat, Chinin-Hydroiodid, Chinin-Hydrochlorid, Chinin-Salicylat oder Chinin-Sulfat.

Die Verbindungen (Prodrugs) verleihen den Arzneistoffen zusätzlich zu ihren pharmokodynamischen Eigenschaften die Fähigkeit, sich in den roten Blutkörperchen (Erythrozyten) anzureichern. Dies führt zu folgenden Therapie-relevanten Eigenschaften bzw. Vorteilen:
1. Körpereigene Substanzen, Naturstoffe und synthetische Substanzen mit therapeutisch wertvollen Eigenschaften mit hohem "first pass" Effekt werden durch die beschriebene Modifikation überhaupt erst sinnvoll oral verfügbar bzw. bezüglich oraler Aktivität entscheidend verbessert. Die Aufnahme der erfindungsgemäßen Substanzen in den Erythrozyten verhindert ihre Extraktion aus dem Blut während der (ersten) Leberpassage und damit ihre Verstoffwechselung und Ausscheidung.
2. Die erfindungsgemäße Modifikation führt zu einer Reduktion unerwünschter Effekte dieser Substanzen in der Leber im Vergleich zur unsubstituierten Ausgangssubstanz.
3. Die Anreicherung in Erythrozyten um ein Vielfaches der Plasmakonzentration kann besonders dann eingesetzt werden, wenn die Wirkung im Erythrozyten Ziel der Therapie ist. Dies ist bei Parasitenbefall der Erythrozyten, zum Beispiel bei Malaria der Fall.
4. Fast alle körpereigenen Hormone, Transmitter-Substanzen und viele Wirkstoffe sind in ihrer therapeutischen Anwendbarkeit durch zu rasche Verstoffwechselung und Eliminierung eingeschränkt. Die erfindungsgemäße Modifikation führt über ein in den Erythrozyten entstehendes Depot zu einer entscheidenden Verlängerung der Verweildauer der wirksamen Substanz im Organismus. Das Auftreten sehr hoher Spiegel im Plasma kurz nach der Applikation wird vermieden. Dies ist ein Vorteil, besonders bei allen Substanzen, die Wirkungen im zentralen und peripheren Nervensystem sowie auf Blutgefäße entfalten.
5. Die vorliegende Erfindung beschreibt u.a. Prodrugs. Die Prodrugs werden an die Erythrozyten gebunden. Die therapeutisch erwünschte Wirkung erfolgt durch die (hydrolytische) Freisetzung des Wirkstoffs aus seinem Prodrug.

Grundsätzlich sind die erfindungsgemäßen Verbindungen in Verbindung mit transkutaner Applikation, Inhalation oder Injektion einsetzbar. Die Therapie-relevanten Eigenschaften bzw. Vorteile der erfindungsgemäßen Prodrugs kommen jedoch insbesondere bei der oralen Anwendung zum Tragen.

Der Erfolg einer Pharmakotherapie hängt naturgemäß von den pharmakodynamischen und pharmakokinetischen Eigenschaften der jeweiligen Therapeutika entscheidend ab. Daneben ist für den Therapieerfolg die Form der Darreichung des Arzneimittels entscheidend. Diese hat entscheidenden Einfluß auf die Anwendbarkeit eines Wirkprinzips durch Arzt oder Patient und die "Compliance" des Patienten. Es kann als gesichert gelten, daß eine orale Therapieform eine bessere "Compliance" gewährleistet als jede andere Form der Verabreichung.

Ein Grund für die hohe Akzeptanz der oralen Applikation ist einmal die Einfachheit der Anwendung und die gute Steuerbarkeit einer Therapie. Ein anderer wesentlicher Grund für die überragende "Compliance" der oralen Applikation dürfte dadurch bedingt sein, daß wir gewohnt sind, Nahrung, Flüssigkeit und Genußmittel durch den Mund aufzunehmen. Andere Applikations-Verfahren können dagegen als Verletzung oder nicht natürlich empfunden werden und sind dadurch besonders im Hinblick auf länger dauernde Therapieformen belastet.
Figur 1 zeigt die Verteilung der Gesamt-Radioaktivität von ³H/¹⁴C-J 1045 vs ³H-J 995 (beide Verbindungen nicht Gegenstand der Erfindung) nach einmaliger oraler Verabreichung.
Figur 2 erläutert das erfindungsgemäße Prinzip anhand J995. (nicht Gegenstand der Erfindung)
Figur 3 zeigt die chemische Synthese eines verknüpfbaren Estradiolsulfamats (J1242) (nicht Gegenstand der Erfindung) und die Immobilisierung dieses Liganden.
Figuren 4 und 5 zeigen Analysen einer Affinitätschromatographie-Fraktion in Western Blots.

### Funktionelle Gruppen

Die funktionelle Gruppe kann grundsätzlich jede Gruppe sein, über die, mittels einem Spacer eine Gruppe -SO₂NR¹R² eingeführt werden kann.

Wie bereits vorstehend ausgeführt weisen die erfindungsgemäßen Verbindungen bevorzugt die folgende Struktur: 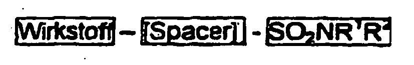 auf, wobei der 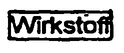 in der freien Form eine 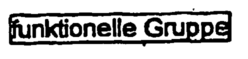 aufweist.

Die funktionelle Gruppe kann eine Gruppe - COOH (oder eine davon abgeleitete Gruppe, wie eine Estergruppe sein) sein, die zusammen mit dem Spacer und mit der Gruppe - SO₂NH₂ eine Gruppe - C(O) - Spacer - SO₂NH₂ bildet.

Die funktionelle Gruppe kann des weiteren eine Gruppe - YH sein, die zusammen mit dem Spacer und mit der Gruppe - SO₂NH₂ eine Gruppe - Y - Spacer -SO₂NH₂ bildet, wobei Y S, O oder NR⁴ bedeutet, wobei R⁴ ein Wasserstoffatom oder eine Alkylgruppe, bevorzugt mit 1 bis 4 Kohlenstoffatomen, ist oder NR⁴ Teil eines heterocyclischen Ringsystems ist

### Wirkstoffe

Der Wirkstoff kann grundsätzlich jeder Wirkstoff sein, der eine funktionelle Gruppe aufweist und mittels dieser über einen Spacer, an die Gruppe - SO₂NR¹R² gebunden werden kann.

Das erfindungsgemäße Prinzip eignet sich besonders für körpereigene Substanzen, Naturstoffe und synthetische Substanzen mit therapeutisch wertvollen Eigenschaften, die einen hohen "first pass" Effekt aufweisen. Diese werden durch die beschriebene Modifikation überhaupt erst sinnvoll oral verfügbar bzw. bezüglich oraler Aktivität entscheidend verbessert.

Insbesondere eignet sich das erfindungsgemäße Prinzip für folgende Wirkstoffklassen bzw. Wirkstoffe:
Hormone, die durch das nachfolgend aufgeführte Grundgerüst beschrieben werden können und mindestens eine funktionelle Gruppe, bevorzugt eine der vorstehend genannten funktionellen Gruppen, im Molekül aufweisen, an die die Gruppe SO₂NR¹R² 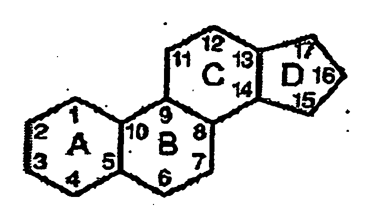 gekoppelt werden kann.

Anabolika bzw. Androgene, die, bevorzugt in 3- oder 17-Stellung, eine Gruppe HO- oder C=O aufweisen, wobei Beispiele für Anabolika Nandrolon, Mentenolon und Trenbolon (funktionelle Gruppe: C=O oder OH) sowie Prasteron sind (funktionelle Gruppe: C=O oder OH) und Beispiele für Androgene Mesterolon, Dehydroepiandrosteron und Testosteron sind (funktionelle Gruppe: C=O oder OH).

Antiandrogene, wie Casodex® (funktionelle Gruppe: OH) und Cyproteron bzw. Cyproteron-Acetat (funktionelle Gruppe: OH, C=O).

Gestagene, die, bevorzugt in 3- oder 17-Stellung, eine Gruppe HO- bzw. C=O aufweisen, wobei Beispiele Dienogest, Levonorgestrel, Hydroxyprogesteron, Norethisteron sowie Gestagene, die sich von Ethinylestradiol oder 17α-Hydroxyprogesteron oder 19-Nortestosteron ableiten (funktionelle Gruppe: 17-OH, 3-C=O), und Drospirenon sind (funktionelle Gruppe: C=O).

Estrogene, die bevorzugt als funktionelle Gruppe eine 3-, 16- oder 17-Hydroxygruppe und/oder 17-Carbonylgruppe aufweisen, wie Estradiol, Estriol oder Estron (funktionelle Gruppe: OH-Gruppe, C=O-Gruppe).

Glucocorticoide, wie Cortison, Dexamethason und Prednisolon (funktionelle Gruppe: OH, C=O).

Amöbizide, wie Metronidazol, Chlortetracyclin, Oxytetracyclin, Demecyclin und Tetracyclin (funktionelle Gruppe: OH) sowie Chlorochin (funktionelle Gruppe: NH).

Anti-Diuretika, wie Vasopressin, Desmopressin, Felypressin, Lypressin, Omipressin und Terlipressin (funktionelle Gruppe: NH).

Antigonadotropine, wie Danazol und Paroxypropion (funktionelle Gruppe: OH).

Ulcus-Therapeutika, wie das Prostaglandin Misoprostol (funktionelle Gruppe: OH).

Dopamin-Receptor-Agonisten, wie Dopexamin, Lisurid und Pergolid (funktionelle Gruppe: OH und/oder NH) sowie Dopamin (funktionelle Gruppe: OH).

Neuropharmaka und Mittel gegen Krebs.

Peptide, wie GnRH und andere hypothalamisch, regulatorisch aktive Peptide.

Antimalariamittel, wie Arteether, Artemether, Artemisinin, Artesunat (funktionelle Gruppe: OH, C=O), Chloroquin, Pamaquin, Primaquin, Pyrethamin (funktionelle Gruppe: NH), Mefloquin (funktionelle Gruppe: NH, OH), Proguanil (funktionelle Gruppe: C=NH), Chinchonidin, Cinchonin (funktionelle Gruppe: OH), Hydroxychloroquin (funktionelle Gruppe: OH, NH), Pamaquin, Primaquin, Pyrimethamin (funktionelle Gruppe: NH) sowie Chinin und Chin-Derivate, wie Chinin-Bisulfat, Chinin-Carbonat, Chinin-Dihydrobromid, Chinin-Dihydrochlorid, Chinin-Ethylcarbonat, Chinin-Format, Chinin-Gluconat, Chinin-Hydroiodid, Chinin-Hydrochlorid, Chinin-Salicylat und Chinin-Sulfat (funktionelle Gruppe: OH).

### Spacer

Erfindungsgemäß wird unter dem Begriff "Spacer" eine Kohlenstoffkette und/oder eine oder mehrere Arylgruppen zwischen der Gruppe - SO₂NHR und der funktionellen Gruppe des Wirkstoffs verstanden.

Aus Gründen der Bioverfügbarkeit und des Transports durch die Membranen weisen die erfindungsgemäßen Verbindungen vorzugsweise ein Molekulargewicht unter 600 auf.

Wenn die funktionelle Gruppe eine Gruppe - COOH ist, ist der Spacer bevorzugt eine Gruppe - A - B - (O)ₛ-, wobei s eine Zahl 0 oder 1 ist, A für S, O oder NR³ steht, wobei R³ ein Wasserstoffatom, eine Alkylgruppe, bevorzugt mit 1 bis 4 Kohlenstoffatomen, oder eine Acylgruppe, bevorzugt mit 1 bis 4 Kohlenstoffatomen, ist, und B aus einer Alkylengruppe, einer Arylengruppe, einer Alkylenarylengruppe oder einer Alkylenarylenalkylengruppe ausgewählt ist, die gegebenenfalls substituiert sind.

Wenn die funktionelle Gruppe eine Gruppe - YH ist, ist der Spacer bevorzugt eine Gruppe 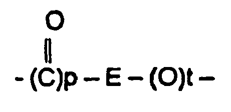 wobei t und p eine Zahl 0 oder 1 sind und E aus einer Alkylengruppe, einer Arylengruppe, einer Alkylenarylengruppe oder einer Alkylenarylenalkylengruppe ausgewählt ist, die gegebenenfalls substituiert sind, oder
eine Gruppe 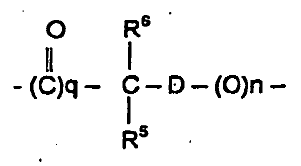 wobei q und n eine Zahl 0 oder 1 sind, R⁵ und R⁶ unabhängig voneinander ein Wasserstoffatom oder eine Alkylgruppe, bevorzugt mit 1 bis 4 Kohlenstoffatomen, bedeuten und D eine Arylengruppe, insbesondere eine Phenylengruppe, bedeutet, die gegebenenfalls substituiert sein kann, oder eine Gruppe 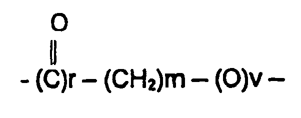 wobei r und v eine Zahl 0 oder 1 sind und m eine Zahl von 1 bis 15 bedeutet.

Unter Alkylengruppe wird erfindungsgemäß eine verzweigte oder unverzweigte Alkylengruppe mit 1 (bzw. 2) bis 20, vorzugsweise 1 (bzw. 2) bis 10 Kohlenstoffatomen verstanden, die gegebenenfalls substituiert sein kann. Der oder die Substituenten können aus C₁₋₄-Alkylgruppen, C₁₋₄-Alkoxygruppen, C₁₋₄-Acylgruppen, Halogenatomen, wie einem Fluor-, Chlor-, Brom- oder lodatomen, einer Hydroxygruppe oder einer Carbonylgruppe ausgewählt werden. Beispiele für eine Alkylengruppe sind Methylen-, Ethylen-, Propylen-, Butylen, Pentylen-, Hexylen-, Heptylen-, Octylen, Nonylen-, Decylen- und Undecylengruppen, die verzweigt oder unverzweigt sein können und gegebenenfalls mit einem oder mehreren der vorstehend genannten Substituenten substituiert sein können.

Der Begriff Alkylengruppe umfaßt erfindungsgemäß auch eine verzweigte oder unverzweigte Alkenylengruppe und Alkinylengruppe mit 2 (bzw. 3) bis 20, vorzugsweise 2 (bzw. 3) bis 10 Kohlenstoffatomen, die gegebenenfalls mit den vorstehend genannten Substituenten substituiert sein können. Beispiele für eine Alkenylen- bzw. Alkinylengruppe leiten sich von den vorstehend für die Alkylengruppe gegebenen Beispielen ab, wobei eine oder mehrere Doppel- bzw. Dreifachbindungen (bis zu 4) vorhanden sein können.

Der Begriff Alkylengruppe umfaßt erfindungsgemäß auch eine Cycloalkylengruppe mit 5 bis 20, vorzugsweise 5 bis 10 Kohlenstoffatomen, die gegebenenfalls substituiert sein kann. Beispiele sind eine Cyclopentylen- oder Cyclohexylengruppe.

Unter einer Arylengruppe wird erfindungsgemäß eine Arylengruppe mit 6 bis 20 Kohlenstoffatomen verstanden, die gegebenenfalls substituiert sein kann. Der oder die Substituenten können aus Halogenatomen, wie einem Fluor-, Chlor-, Brom- oder lodatom, einer Hydroxygruppe, einer Aminogruppe, einer Nitrogruppe, einer C₁₋₄ Alkoxygruppe Acylgruppe oder einer C₁₋₄-Alkylgruppe ausgewählt werden. Beispiele für eine Arylengruppe sind eine Phenylengruppe, eine Halogenphenylengruppe, eine Hydroxyphenylengruppe oder eine Naphthylengruppe, wobei eine Phenylengruppe bevorzugt ist

Unter einer Arylengruppe wird erfindungsgemäß auch eine Heteroarylengruppe mit 5 bis 20 Kohlenstoffatomen verstanden, wobei 1 bis 3 Kohlenstoffatome durch ein Schwefelatom, ein Sauerstoffatom oder ein Stickstoffatom ersetzt sein können. Beispiele für eine Heteroarylengruppe leiten sich von einem Pyridin-, Furan-, Thiophen-, Pyrrol-, Imidazol-, Pyrazin-, Pyrimidin oder Pyridazinring ab.

Unter einer Alkylenarylengruppe wird erfindungsgemäß eine unverzweigte oder verzweigte Alkylengruppe mit 1 bis 14 Kohlenstoffatomen verstanden, der mit einer Arylengruppe mit 6 bis 19 Kohlenstoffatomen verbunden ist. Für beide Reste gelten mit Ausnahme der Anzahl der Kohlenstoffatome die bereits vorstehend für die Alkylengruppe bzw. Arylengruppe gegebenen Definitionen. Beispiele für eine Alkylenarylengruppe sind eine Benzylidengruppe bzw. eine substituierte Benzylidengruppe, in der eines oder beide Wasserstoffatome der Methylengruppe gegen eine C₁₋₄-Alkylgruppe ausgetauscht sind.

Unter einer Alkylenarylenalkylengruppe wird erfindungsgemäß eine verzweigte oder unverzweigte Alkylengruppe mit 1 bis 13 Kohlenstoffatomen verstanden, die mit einer Arylengruppe mit 6 bis 18 Kohlenstoffatomen verbunden ist, die wiederum mit einer unverzweigten oder verzweigten Alkylengruppe mit 1 bis 13 Kohlenstoffatomen verbunden ist. Für beide Arten von Gruppen gelten mit Ausnahme der Anzahl der Kohlenstoffatome die bereits vorstehend für die Alkylengruppe bzw. Arylengruppe gegebenen Definitionen. Bevorzugt ist eine Gruppe 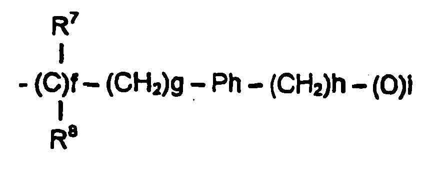 wobei f und i eine Zahl 0 oder 1 sind, g und h eine Zahl von 1 bis 5 bedeuten und R⁷ und R⁸ unabhängig voneinander ein Wasserstoffatom oder eine C₁₋₄-Alkylgruppe bedeuten.

Beispiele für eine Alkylgruppe mit 1 bis 4 Kohlenstoffatomen sind eine Methyl-, Ethyl-, Propyl-, iso-Propyl, Butyl- oder tert.-Butylgruppe.

Beispiele für eine Alkoxygruppe mit 1 bis 4 Kohlenstoffatomen sind eine Methoxy,-Ethoxy-, Propoxy-, Isopropoxy- und tert.-Butyloxygruppe.

Beispiele für eine C₁₋₄-Acylgruppe sind eine Acetylgruppe, Propionylgruppe oder Butyrylgruppe.

Unter Halogenatom wird in der vorliegenden Erfindung ein Fluor-, Chlor, Brom- oder lodatom verstanden.

### Pharmazeutische Zusammensetzungen

Gegenstand der Erfindung sind auch pharmazeutische Zusammensetzungen, die mindestens eine erfindungsgemäße Verbindung zusammen mit pharmazeutisch verträglichen Hilfs- und/oder Trägerstoffen enthalten.

Arzneimittel der vorliegenden Erfindung werden mit den üblichen festen oder flüssigen Trägerstoffen und/oder Verdünnungsmitteln und den allgemein üblicherweise verwendeten Hilfsstoffen entsprechend der gewünschten Applikationsart in einer geeigneten Dosierung und in an sich bekannter Weise hergestellt. Bei der bevorzugten oralen Darreichungsform werden vorzugsweise Tabletten, Filmtabletten, Dragees, Kapseln, Pillen, Pulver, Lösungen oder Suspensionen auch als Depotform zubereitet.

Arzneiformen als Tabletten können beispielsweise durch Mischen des Wirkstoffes mit bekannten Hilfsstoffen, wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk erhalten werden. Die Tabletten können auch aus mehreren Schichten bestehen oder eine Bruchkerbe enthalten.

Weiterhin lassen sich Dragees durch Überziehen von analog den Tabletten hergestellten Kemen mit üblicherweise in Drageeüberzügen verwendeten Mitteln, beispielsweise Polyvinylpyrrolidon oder Schellack, Gummiarabicum, Talk, Titandioxid, oder Zucker bereiten. Die Drageehülle kann dabei auch aus mehreren Schichten bestehen, wobei beispielsweise oben genannte Hilfsstoffe verwendet werden.

Lösungen oder Suspensionen mit dem erfindungsgemäßen Wirkstoff können zur Verbesserung des Geschmacks mit Stoffen wie Saccharin, Cyclamat oder Zucker und /oder mit Aromastoffen, wie Vanillin oder Orangenextrakt versetzt werden. Weiterhin können sie mit Suspendierhilfsstoffen, wie Natriumcarboxymethylcellulose, oder Konservierungsmitteln, wie p-Hydroxybenzoesäure, vermischt werden.

Die Bereitung von Kapseln kann durch Mischen des Arzneistoffes mit Trägem wie Milchzucker oder Sorbit erfolgen, die dann in die Kapseln eingebracht werden.

Die Herstellung von Suppositorien erfolgt vorzugsweise durch Mischen des Wirkstoffes mit geeigneten Trägermaterialien wie Neutraffetten oder Polyethylenglykolen oder dessen Derivaten.

Aus dem Depoteffekt ergibt sich eine hohe orale Bioverfügbarkeit des Wirkstoffs, was einerseits sehr niedrige Dosierungen des Sulfamats und andererseits die Anwendung in größeren Intervallen erlaubt.

Das heißt, die Dosierungen für die erfindungsgemäßen Verbindungen, wenn sie als Prodrug eingesetzt werden, können bei oraler Verabreichung sehr viel niedriger liegen als bei Verabreichung des im Prodrug enthaltenen Wirkstoffs selbst, und der Wirkstoff kann in größeren Intervallen verabreicht werden.

Für J995 (nicht Gegenstand der Erfindung) wurde beispielsweise gefunden, daß die einzelne Dosierungseinheit 20 - 300 µg Estrogensulfamat für einen Applikationsabstand von 1 bis 3 Tagen, 0,5 - 5,0 mg Estrogensulfamat für einen Applikationsabstand von 5 bis 10 Tagen oder 2,0 - 20 mg Estrogensulfamat für einen Applikationsabstand von 20 bis 40 Tagen betragen kann.

Die erfindungsgemäßen Verbindungen, Wirkstoffe und Spacer, sind kommerziell verfügbar oder können nach in der Literatur bekannten Verfahren hergestellt werden [siehe beispielsweise S. Schwarz et al., Steroids 61 (1996) 710-717; Pharmazie 30 (1975) 17 ff., WO96/05216, WO97/14712, WO93/05064, DE-A-1203042, Appel, Senkpiel, Chem. Ber. 92 (1959) 1102-1104, G. Weiss, G. Schulze, Justus Liebigs Ann. Chem. 729 (1969) 40-51, M. J. Reed et al., Pharm. Sci. (1996) 11-16, U. G. Sahm et. al., Pharm. Sci. (1996) 17-20].

### Darstellung der Vorteile des erfindungsgemäßen Prinzips:

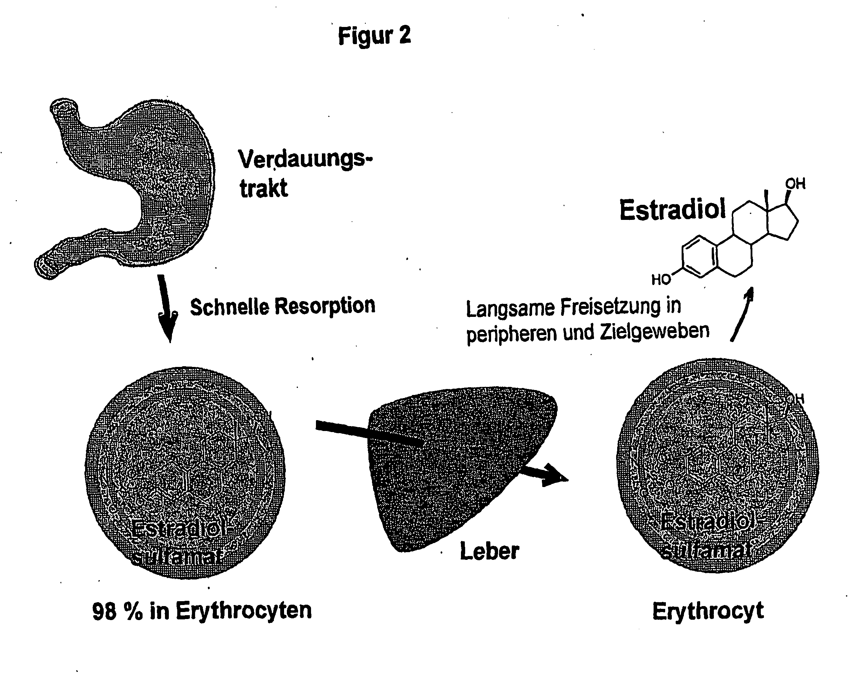

Estrogensulfamate sind Prodrugs von Estrogenen und zeigen von den Estrogenen unterschiedliche pharmakokinetische Eigenschaften nach oraler Verabreichung, vergleichbar Kinetiken die bei transdermaler Verabreichung erreicht werden. Nach oraler Verabreichung wird Estradiolsulfamat vom Magen-Darm-Trakt resorbiert und wird in die Erythrozyten aufgenommen bzw. an die Erythrozyten gebunden. Durch die Aufnahme in die Erythrozyten bzw. durch die Bindung an die Erythrozyten wird die Extraktion des Sulfamats aus dem Blut während der (ersten) Leberpassage und damit seine Verstoffwechselung und Ausscheidung verhindert, wodurch das Estradiolsulfamat u. a. eine reduzierte hepatische Estrogenität zeigt. Das Estradiolsulfamat wird in den Erythrozyten um ein Vielfaches (Faktor: 98:2) der Plasmakonzentration angereichert, wobei ein Depot des Estradiolsulfamats entsteht, das die langsame Freisetzung des Estradiolsulfamats in peripheren Geweben und Zielgeweben ermöglicht (siehe Figur 2). Dies ermöglicht eine Verlängerung der Verweildauer des Estradiolsulfamats im Organismus und das Auftreten sehr hoher Spiegel im Plasma wird vermieden.

Kennzeichnend für das Estradiolsulfamat sind folglich:
(a) Aufnahme des Prodrugs in bzw. Bindung des Prodrugs an die Erythrozyten und
(b) Anreicherung des Prodrugs in den Erythrozyten um ein Vielfaches der Plasmakonzentration (Depoteffekt)
(c) langsame Freisetzung des Wirkstoffs aus dem Depot.

Daraus ergeben sich die folgenden Vorteile:
- Sehr gleichmäßige und lang anhaltende Wirkstoffspiegel im Blut aufgrund des Depoteffekts,
- hohe orale Bioverfügbarkeit des Wirkstoffs, was einerseits sehr niedrige Dosierungen des Sulfamats und andererseits die Anwendung in größeren Intervallen erlaubt,
- Verringerung der individuellen Variation von Blutspiegeln sowie
- Verringerung der hepatischen estrogenen Wirkung.

### Identifizierung des spezifischen Trägerproteins für Estradiolsulfamat in Erythrozyten

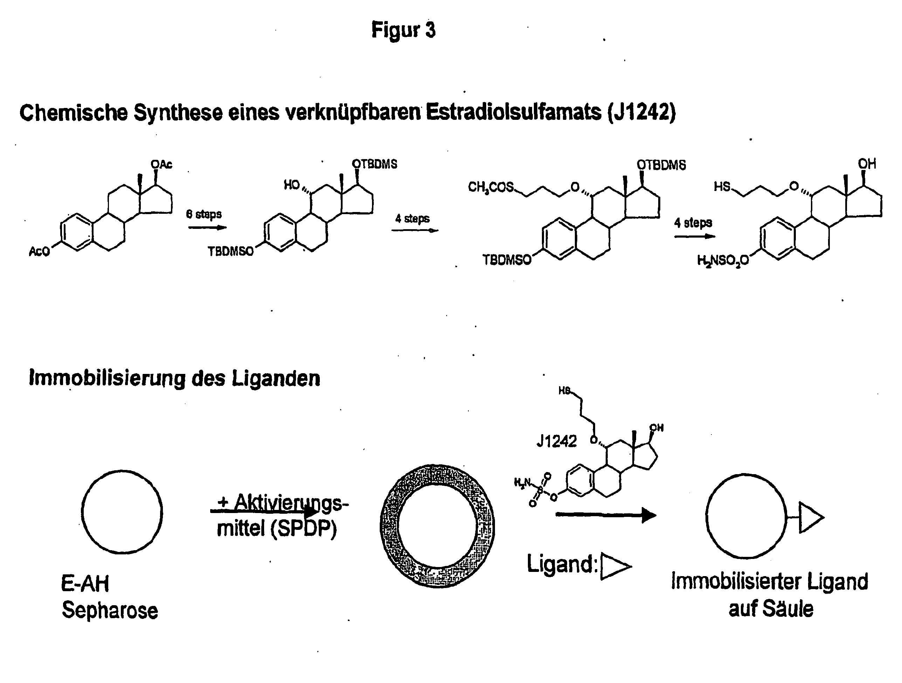

In Figur 3 ist die chemische Synthese eines verknüpfbaren Estradiolsulfarnats (J1242) (nicht Gegenstand der Erfindung) gezeigt, die in S. Schwarz et al., Steroids 64 (1999) 460-471 beschrieben ist.

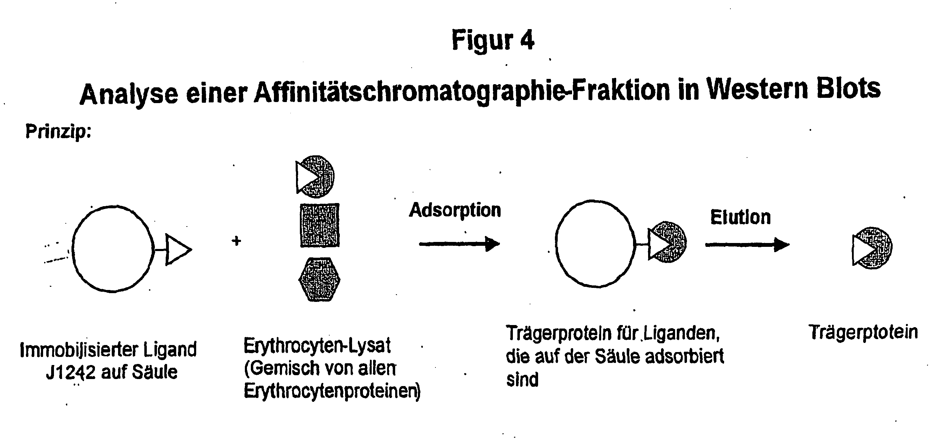

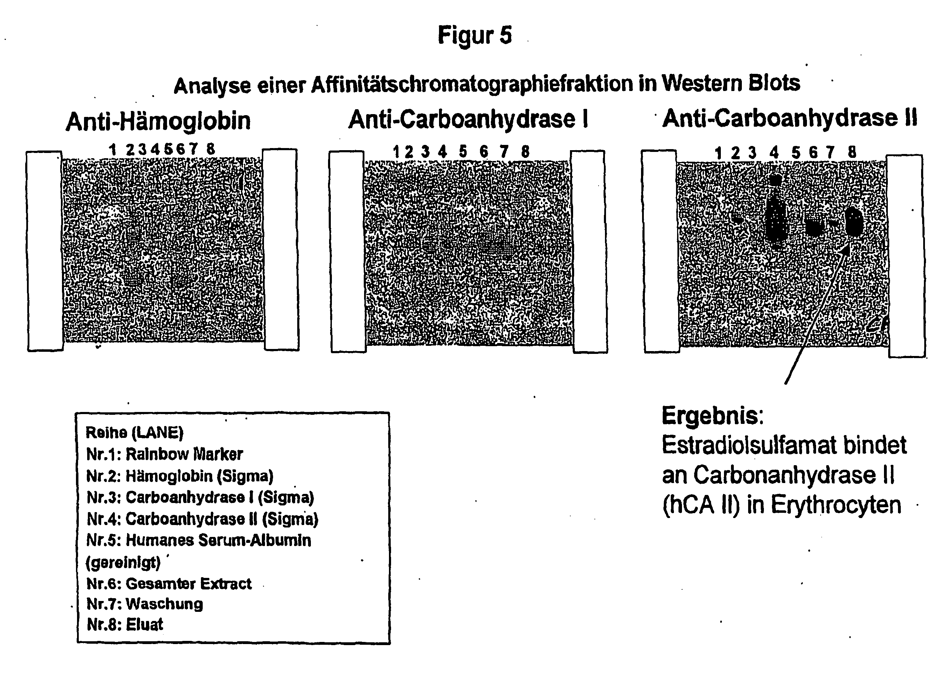

Nach Immobilisierung des Estradiolsulfamatderivates auf dem Säulenmaterial (E-AH-Sepharose) wurden Proteine von Erythrozyten-Lysaten auf der Säule getrennt. Das Protein, das für die Bindung des Estradiolsulfamats in Erythrozyten verantwortlich ist, wurde auf der Oberfläche der Affinitätschromatographiesäule adsorbiert. Nach Waschen und Elution des auf der Säule zurückgebliebenen Proteins mit einem Acetatpuffer wurde das Eluat unter Verwendung der "Western Plot Technique" analysiert (siehe Figuren 4 und 5).
Als Ergebnis wurde gefunden, daß das Estradiolsulfamat in Erythrozyten an die Carboanhydrase II (hCA II) bindet Die Affinitätschromatographie zeigt, daß Proteinbestandteile der Erythrozyten spezifisch gebunden werden, wobei dieses Ergebnis die Bindung von Estradiolsulfamat und von erfindungsgemäßen Substanzen an weitere Proteine in Erythrozyten nicht ausschließt, wenn die Affinität dieser Proteine zu J1242 hinter der hCA II zurückbleibt.

## Patentansprüche

1. Verbindung, die als Prodrug und/oder Träger die Aufnahme eines Wirkstoffs in die Erythrozyten und/oder die Bindung eines Wirkstoffs an die Erythrozyten ermöglicht,
**dadurch gekennzeichnet, dass**
die Aufnahme der Verbindung in und/oder die Bindung der Verbindung an die Erythrozyten durch eine Gruppe
-SOP₂NR¹R²
ermöglicht wird, wobei R¹ und R² unabhängig voneinander ein Wasserstoffatom, ein Acylrest, ein Alkylrest, ein Cycloalkylrest, ein Arylrest, eine Cyanogruppe oder eine Hydroxygruppe sind und wobei die Verbindung ein Prodrug ist, das die folgende Struktur aufweist:
Wirkstoff - Spacer -SO₂NR¹R²,
wobei der Wirkstoff in seiner freien Form eine funktionelle Gruppe aufweist, zur pharmazeutischen Verwendung.

2. Verbindung nach Anspruch 1, wobei die Aufnahme der Verbindung in und/oder die Bindung, der Verbindung an die Erythrozyten über Hämoglobin, Membranproteine und/oder Carboanhydrase erfolgt.

3. Verbindung nach Anspruch 1 und/oder 2, wobei durch die Aufnahme der Verbindung in und/oder durch die Bindung der Verbindung an die Erythrozyten ein Depot des Wirkstoffs in den Erythrozyten gebildet wird, wobei ein wesentlicher Teil des Wirkstoffs im Körper in Erythrozyten vorliegt.

4. Verbindung nach Anspruch 3, wobei die Verbindung in den Erythrozyten um den Faktor 10 bis 1000 über Plasmaspiegel angereichert wird.

5. Verbindung nach einem der Ansprüche 1 bis 4, wobei es sich bei der Verbindung um eine Verbindung handelt, die in den Erythrozyten ihre Wirkung entfaltet.

6. Verbindung nach Anspruch 1, wobei einer der Reste R¹ und R² ein Wasserstoffatom bedeutet.

7. Verbindung nach Anspruch 1, wobei R¹ und R² ein Wasserstoffatom bedeuten.

8. Verbindung nach einem der Ansprüche 1 bis 7, wobei die Verbindung und/oder der in der Verbindung enthaltene Wirkstoff ein Parasitenbefall der Erythrozyten verhindert.

9. Verbindung nach Anspruch 8, wobei der Wirkstoff ein Anti-Malariamittel, wie Arteether, Artemether, Artesunat, Chloroquin, Primaquin, Pyrethamin, Mefloquin, Proguanil, Chinchonidin, Cinchonin, Hydroxychloroquin, Pamaquin, Primaquin, Pyrimetamin, Chinin oder ein Chinin-Derivat, wie Chinin-Bisulfat, Chinin-Carbonat, Chinin-Dihydrobromid, Chinin-Dihydrochlorid, Chinin-Ethylcarbonat, Chinin-Format, Chinin-Gluconat, Chinin-Hydroiodid, Chinin-Hydrochlorid, Chinin=Salicylat oder Chinin-Sulfat, ist.

10. Verbindung nach einem der Ansprüche 1 bis 9, wobei die therapeutisch erwünschte Wirkung durch Freisetzung, insbesondere hydrolytische Spaltung des im Prodrug enthaltenen Wirkstoffs und/oder seiner Metabolite erfolgt.

11. Verbindung nach Anspruch 1, wobei die funktionelle Gruppe eine Gruppe -COOH ist, die zusammen mit dem Spacer und mit der Gruppe SO₂NH₂ eine Gruppe -C (O)- Spacer - SO₂NH₂ bildet.

12. Verbindung nach Anspruch 10, wobei der Spacer eine Gruppe -A-B-(O)ₛ- ist, wobei s eine Zahl 0 oder 1 ist, A für S, O oder NR³ steht, wobei R³ ein Wasserstoffatom, ein Alkylrest oder ein Acylrest ist, und B aus einer Alkylengruppe, einer Arylengruppe, einer Alkylenarylgruppe, oder einer Alkylenarylenalkylgruppe ausgewählt ist, die gegebenenfalls substituiert sind.

13. Verbindung nach Anspruch '7, wobei die funktionelle Gruppe eine Gruppe -YH ist, die zusammen mit dem Spacer und mit der Gruppe - SO₂NH₂ eine Gruppe -Y-Spacer - SO₂NH₂ bildet, wobei Y für S, O oder NR⁴ steht, wobei R⁴ ein Wasserstoffatom, ein Alkylrest oder ein Acylrest ist oder NR⁴ Teil eines heterocyclischen Ringsystems ist.

14. Verbindung nach Anspruch 13, wobei der Spacer eine Gruppe 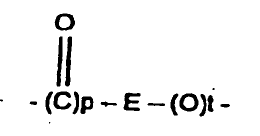 ist, wobei t und p eine Zahl 0 oder 1 sind und E aus einer Alkylengruppe, einer Arylengruppe, einer Alkylenarylgruppe oder einer Alkylenarylenalkylgruppe ausgewählt ist, die gegebenenfalls substituiert sind.

15. Verbindung nach Anspruch 13, wobei der Spacer eine Gruppe 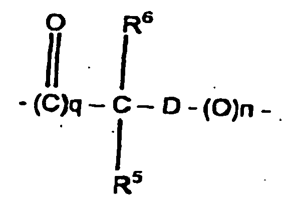 bedeutet, wobei q und n eine Zahl 0 oder 1 sind, R⁵ und R⁶ unabhängig voneinander ein Wasserstoffatom oder einen Alkylrest bedeuten und D eine Arylengruppe, insbesondere eine Phenylengruppe, bedeutet, die gegebenenfalls substituiert sein kann.

16. Verbindung nach Anspruch 11, wobei der Spacer eine Gruppe 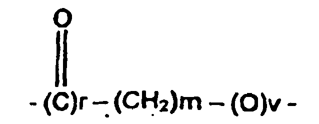 bedeutet, wobei r und v eine Zahl 0 oder 1 sind und m eine Zahl von 1 bis 15 bedeutet.

17. Verbindung nach einem der Ansprüche 14 bis 16, wobei die funktionelle Gruppe eine Gruppe -OH ist.

18. Verbindung nach einem der Ansprüche 1, 6-17, wobei der Wirkstoff aus Androgenen, Anabolika, Antiandrogen, Estrogenen, Gestagenen, Gluccocorticoiden, Amöbiziden, Anti-Diuretika, Antigonatropinen, Ulcus-Therapeutika, Neuropharmaka, Dopaminrezeptorantagonisten, Dopaminrezeptoragonisten, Dopamin, Apomorphin, Melatonin und Peptiden, wie GnRH und anderen hypothalamisch, regulatorisch aktiven Peptiden, ausgewählt ist.

19. Verbindung nach Anspruch 18, wobei der Wirkstoff ein Androgen ist und die funktionelle Gruppe die 17-Hydroxygruppe oder die 3-Carbonylgruppe des Androgens ist.

20. Verbindung nach Anspruch 19, wobei das Androgen Testosteron ist.

21. Verbindung nach Anspruch 18, wobei der Wirkstoff Estrogen ist und die funktionelle Gruppe eine 3-, 16 oder 17-Hydroxygruppe oder 17-Carbonylgruppe ist.

22. Verbindung nach Anspruch 21, wobei der Wirkstoff aus Estradiol, Estriol oder Estron ausgewählt ist.

23. Verbindung nach Anspruch 18, wobei der Wirkstoff ein Gestagen ist und die funktionelle Gruppe eine 17-Hydroxygruppe oder eine 3-Carbonylgruppe ist.

24. Verbindung nach Anspruch 23, wobei der Wirkstoff aus Norethisteron, Dienogest, Drospirenon oder Levomorgestrel ausgewählt ist.

25. Verwendung einer Verbindung, wie in einem der Ansprüche 1 bis 24 definiert, zur Herstellung eines depotbildenden Arzneimittels, die als Prodrug und/oder Träger die Aufnahme eines Wirkstoffs in die Erythrozyten und/oder die Bindung eines Wirkstoffs an die Erythrozyten ermöglicht, wobei die Aufnahme der Verbindung in und/oder die Bindung der Verbindung an die Erythrozyten durch eine Gruppe
-SO₂NR¹R²
ermöglicht wird, und wobei R¹ und R² unabhängig voneinander ein Wasserstoffatom, ein Acylrest, ein Alkylrest, ein Cycloalkylrest, ein Arylrest, eine Cyanogruppe oder eine Hydroxygruppe sind und wobei die Verbindung ein Prodrug ist, das die folgende Struktur aufweist:
Wirkstoff - Spacer - SO₂NR¹R²
wobei der Wirkstoff in seiner freien Form eine funktionelle Gruppe aufweist.

## Claims

1. Compound that as a prodrug and/or vehicle makes it possible to take up an active ingredient into erythrocytes and/or to bind an active ingredient to erythrocytes, **characterized in that** the uptake of the compound into erythrocytes and/or the binding of the compound to erythrocytes is made possible by a group
-SO₂NR¹R²
whereby R¹ and R², independently of one another, is a hydrogen atom, an acyl radical, an alkyl radical, a cycloalkyl radical, an aryl radical, a cyano group or a hydroxy group and wherein the compound is a prodrug that has the following structure:
Active ingredient -Spacer - SO₂NR¹R²
whereby the active ingredient in its free form has a functional group, for pharmaceutical use.

2. Compound according to Claim 1, whereby the uptake of the compound into erythrocytes and/or the binding of the compound to erythrocytes is carried out via haemoglobin, membrane proteins and/or carboanhydrase.

3. Compound according to Claim 1 and/or 2, whereby a depot of the active ingredient in the erythrocytes is formed by the uptake of the compound into erythrocytes and/or by the binding of the compound to erythrocytes, whereby an essential part of the active ingredient is present in the body in erythrocytes.

4. Compound according to Claim 3, whereby the compound is enriched in the erythrocytes by a factor 10 to 1000 above the plasma level.

5. Compound according to one of Claims 1 to 4, whereby the compound is a compound that exerts its action in the erythrocytes.

6. Compound according to Claim 1, whereby one of radicals R¹ and R² means a hydrogen atom.

7. Compound according to Claim 1, whereby R¹ and R² mean a hydrogen atom.

8. Compound according to one of Claims 1 to 7, whereby the compound and/or the active ingredient that is contained in the compound prevents the parasitic attack of the erythrocytes.

9. Compound according to Claim 8, whereby the active ingredient is an anti-malaria agent, such as arteether, artemether, artesunate, chloroquine, primaquine, pyrethamine, mefloquine, proguanil, cinchonidine, cinchonine, hydroxychloroquine, pamaquine, primaquine, pyrimethamine, quinine or a quinine derivative, such as quinine-bisulphate, quinine-carbonate, quinine-dihydrobromide, quinine-dihydrochloride, quinine-ethylcarbonate, quinine-formate, quinine-gluconate, quinine-hydroiodide, quinine-hydrochloride, quinine-salicylate or quinine-sulphate.

10. Compound according to one of Claims 1 to 9, whereby the therapeutically desired action is carried out by release, especially hydrolytic cleavage, of the active ingredient that is contained in the prodrug and/or its metabolites.

11. Compound according to Claim 1, whereby the functional group is a group -COOH, which together with the spacer and with the group SO₂NH₂ forms a group -C(O)-spacer-SO₂NH₂.

12. Compound according to Claim 10, whereby the spacer is a group -A-B- (O)ₛ-, whereby s is a number 0 or 1, A stands for S, 0 or NR³, whereby R³ is a hydrogen atom, an alkyl radical or an acyl radical, and B is selected from an alkylene group, an arylene group, an alkylene aryl group or an alkylene arylenealkyl group, which optionally are substituted.

13. Compound according to Claim 7, whereby the functional group is a group -YH, which together with the spacer and with the group -SO₂NH₂ forms a group -Y-spacer-SO₂NH₂, whereby Y stands for S, O or NR⁴, whereby R⁴ is a hydrogen atom, an alkyl radical or an acyl radical, or NR⁴ is part of a heterocyclic ring system.

14. Compound according to Claim 13, whereby the spacer is a group 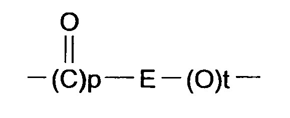 whereby t and p are a number 0 or 1, and E is selected from an alkylene group, an arylene group, an alkylene aryl group or an alkylene arylenealkyl group, which optionally are substituted.

15. Compound according to Claim 13, whereby the spacer means a group 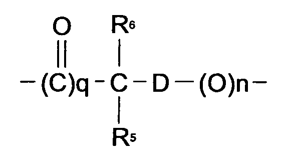 whereby q and n are a number 0 or 1, R⁵ and R⁶, independently of one another, mean a hydrogen atom or an alkyl radical, and D means an arylene group, especially a phenylene group, which optionally can be substituted.

16. Compound according to Claim 11, whereby the spacer means a group 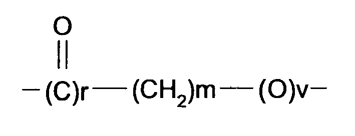 whereby r and v are a number 0 or 1, and m means a number from 1 to 15.

17. Compound according to one of Claims 14 to 16, whereby the functional group is a group -OH.

18. Compound according to one of Claims 1, 6-17, whereby the active ingredient is selected from androgens, anabolic agents, antiandrogens, oestrogens, gestagens, glucocorticoids, amoebicides, anti-diuretic agents, antigonatropines, ulcer therapeutic agents, neuropharmaceutical agents, dopamine receptor antagonists, dopamine receptor agonists, dopamine, apomorphine, melatonin and peptides, such as GnRH, and other hypothalamic, regulatory active peptides.

19. Compound according to Claim 18, whereby the active ingredient is an androgen, and the functional group is the 17-hydroxy group or the 3-carbonyl group of the androgen.

20. Compound according to Claim 19, whereby the androgen is testosterone.

21. Compound according to Claim 18, whereby the active ingredient is an oestrogen, and the functional group is a 3-, 16- or 17-hydroxy group or 17-carbonyl group.

22. Compound according to Claim 21, whereby the active ingredient is selected from estradiol, estriol or estrone.

23. Compound according to Claim 18, whereby the active ingredient is a gestagen, and the functional group is a 17-hydroxy group or a 3-carbonyl group.

24. Compound according to Claim 23, whereby the active ingredient is selected from norethisterone, dienogest, drospirenone or levonorgestrel.

25. Use of a compound as defined in one of Claims 1 to 24 for the preparation of a depot forming medicament that as a prodrug and/or vehicle makes it possible to take up an active ingredient into erythrocytes and/or to bind an active ingredient to erythrocytes, whereby the uptake of the compound into erythrocytes and/or the binding of the compound to erythrocytes is made possible by a group
-SO₂NR¹R²
and whereby R¹ and R², independently of one another, is a hydrogen atom, an acyl radical, an alkyl radical, a cycloalkyl radical, an aryl radical, a cyano group or a hydroxy group and wherein the compound is a prodrug that has the following structure:
Active ingredient -Spacer - SO₂NR¹R²
whereby the active ingredient in its free form has a functional group.

## Revendications

1. Composé, qui permet, en tant que promédicament et/ ou véhicule l'absorption d'une substance active dans les érythrocytes et/ou la liaison d'une substance active aux érythrocytes,
**caractérisé en ce que**
l'absorption du composé et/ou la liaison aux érythrocytes est rendue possible par un groupement
-SO₂NR¹R²,
dans lequel R¹ et R² sont indépendamment l'un de l'autre un atome d'hydrogène, un groupement acyle, un groupement alkyle, un groupement cycloalkyle, un groupement aryle, un groupement cyano ou un groupement hydroxy et moyennant quoi le composé est un promédicament qui a la constitution suivante :
substance active - espaceur - SO₂NR¹R²,
dans laquelle la substance active sous sa forme libre comporte un groupement fonctionnel, pour usage pharmaceutique.

2. Composé selon la revendication 1, dans lequel l'absorption du composé et/ou la liaison du composé aux érythrocytes s'effectue par l'intermédiaire de l'hémoglobine, des protéines membranaires et/ou de l'anhydrase carbonique.

3. Composé selon la revendication 1 et/ou 2, dans lequel par l'absorption du composé dans les érythrocytes et/ou la liaison du composé aux érythrocytes, un dépôt de la substance active se forme dans les érythrocytes, moyennant quoi une partie importante de la substance active dans le corps se trouve dans les érythrocytes.

4. Composé selon la revendication 3, dans lequel le composé dans les érythrocytes est enrichi d'un facteur de 10 à 1000 au-dessus du niveau de plasma.

5. Composé selon une des revendications 1 à 4, s'agissant d'un composé qui déploie son effet dans les érythrocytes.

6. Composé selon la revendication 1, dans lequel un des groupements R¹ et R² représentent un atome d'hydrogène.

7. Composé selon la revendication 1, dans lequel R¹ et R² représentent un atome d'hydrogène.

8. Composé selon une des revendications 1 à 7, moyennant quoi le composé et/ou la substance active contenue dans le composé empêche une infestation parasitaire des érythrocytes.

9. Composé selon la revendication 8, dans lequel la substance active est un agent anti-malaria tel que de l'artéther, de l'artéméther, de l'artésunate, de la chloroquine, de la primaquine, du pyréthamine, de la méfloquine, du proguanil, de la cinchonidine, de la cinchonine, de l'hydroxychloroquine, de la pamaquine, du pyrimétamine, de la quinine ou un dérivé de quinine comme du quinine-bisulfate, du quinine-carbonate, du quinine-dihydrobromure, du quinine-dichlorhydrate, du quinine-éthylcarbonate, du quinine-formate, du quinine-gluconate, du quinine-hydroiodure, du quinine-chlorhydrate, du quinine-salicylate ou du quinine-sulfate.

10. Composé selon une des revendications 1 à 9, moyennant quoi l'effet thérapeutique souhaité s'effectue par libération, notamment par libération hydrolytique de la substance active contenue dans le promédicament et/ou de sa métabolite.

11. Composé selon la revendication 1, dans lequel le groupement fonctionnel est un groupement -COOH, qui forme ensemble avec l'espaceur et le groupement SO₂NH₂ un groupement -C(O)- espaceur - SO₂NH₂.

12. Composé selon la revendication 10, dans lequel l'espaceur est un groupement -A-B-(O)ₛ-, dans lequel s est un nombre égal à 0 ou 1, A représente S, O ou NR³, dans lequel R³ est un atome d'hydrogène, un groupement alkyle ou un groupement acyle et B est choisi parmi un groupement alkylène, un groupement arylène, un groupement alkylènaryle, ou un groupement alkylènarylènalkyle, qui sont éventuellement substitués.

13. Composé selon la revendication 7, dans lequel le groupement fonctionnel est un groupement -YH, qui forme ensemble avec l'espaceur et le groupement SO₂NH₂ un groupement -Y- espaceur - SO₂NH₂, dans lequel Y représente S, 0 ou NR⁴, dans lequel R⁴ est un atome d'hydrogène, un groupement alkyle ou un groupement acyle ou NR⁴ est une partie d'un système de noyau hétérocyclique.

14. Composé selon la revendication 13, dans lequel l'espaceur est un groupement 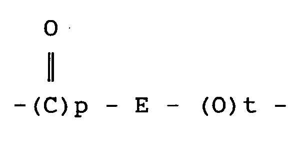 dans lequel p et t sont des nombres égaux à 0 ou 1 et E est choisi parmi un groupement alkylène, un groupement arylène, un groupement alkylènaryle, ou un groupement alkylènarylènalkyle, qui sont éventuellement substitués.

15. Composé selon la revendication 13, dans lequel l'espaceur représente un groupement 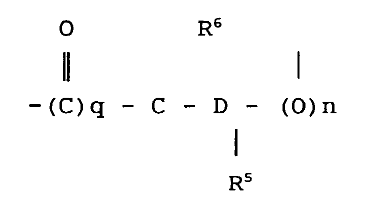 dans lequel q et n sont des nombres égaux à 0 ou 1, R⁵ et R⁶ représentent indépendamment l'un de l'autre un atome d'hydrogène ou un groupement alkyle et D représente un groupement arylène, notamment un groupement phénylène, qui peut éventuellement être substitué.

16. Composé selon la revendication 11, dans lequel l'espaceur est un groupement 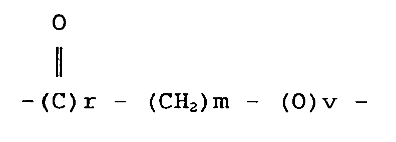 dans lequel r et v sont des nombres égaux à 0 ou 1 et m est un nombre compris entre 1 et 15.

17. Composé selon l'une des revendications 14 à 16,
dans lequel le groupement fonctionnel est un groupement -OH.

18. Composé selon l'une des revendications 1, 6 à 17, moyennant quoi la substance active est choisie parmi les androgènes, les anabolisants, les antiandrogènes, les estrogènes, les gestagènes, les glucocorticoïdes, les amibicides, les antidiurétiques, les anti-gonadotropines, les agents thérapeutiques de l'ulcère, les agents neuropharmaceutiques, les antagonistes des récepteurs de dopamine, les agonistes des récepteurs de dopamine, la dopamine, l'apomorphine, la mélatonine et les peptides tels que le GnRH et d'autres peptides ayant une action de régulation hypothalamique.

19. Composé selon la revendication 18, dans lequel la substance active est un androgène et le groupement fonctionnel est le groupement 17-hydroxy ou le groupement 3-carbonyle de l'androgène.

20. Composé selon la revendication 19, dans lequel l'androgène est de la testostérone.

21. Composé selon la revendication 18, dans lequel la substance active est un estrogène et le groupement fonctionnel est un groupement 3-, 16-, ou 17-hydroxy ou un groupement 17-carbonyle.

22. Composé selon la revendication 21, dans lequel la substance active est choisie parmi de l'estradiol, de l'estriol ou de l'estrone.

23. Composé selon la revendication 18, dans lequel la substance active est un gestagène et le groupement fonctionnel est un groupement 17-hydroxy ou un groupement 3-carbonyle.

24. Composé selon la revendication 23, dans lequel la substance active est choisie parmi de la noréthisterone, du diénogeste, du drospirénone ou du lévonorgestrel.

25. Utilisation d'un composé comme défini dans l'une des revendications 1 à 24 pour préparer un médicament à action retard, qui permet, à titre de promédicament et/ou de véhicule l'absorption d'une substance active dans les érythrocytes et/ou la liaison d'une substance active aux érythrocytes, moyennant quoi l'absorption du composé dans et/ou la liaison du composé aux érythrocytes est rendue possible par un groupement
-SO₂NR¹R²
dans lequel R¹ et R² sont indépendamment l'un de l'autre un atome d'hydrogène, un groupement acyle, un groupement alkyle, un groupement cycloalkyle, un groupement aryle, un groupement cyano ou un groupement hydroxy et le composé est un promédicament qui a la constitution suivante :
substance active - espaceur -SO₂NR¹R²,
dans laquelle la substance active comporte dans sa forme libre un groupement fonctionnel.
